# EUROPÄISCHE PATENTANMELDUNG

(11) **EP 1 310 481 A1**
(43) Veröffentlichungstag der Anmeldung: **14.05.2003**
(21) Anmeldenummer: 02023509.9
(22) Anmeldetag: 22.10.2002
(51) Int. Cl.: C07C 231/02

(54) **Verfahren zur Herstellung von N-Formyl-aminocarbonsäureestern**

(30) Priorität: 09.11.2001 DE 10154716
(71) Anmelder: BASF AKTIENGESELLSCHAFT, 67056 Ludwigshafen (DE)
(72) Erfinder: Wartini, Alexander, Dr., 69120 Heidelberg (DE); Bergner, Eike Johannes, Dr., 69198 Schriesheim (DE); Ebel, Klaus, Dr., 68623 Lampertheim (DE)

(57) **Zusammenfassung**

Die Erfindung betrifft ein Verfahren zur Herstellung von N-Formyl-aminocarbonsäureestern durch Umsetzung von Aminocarbonsäuren mit Ameisensäureestern.

## Beschreibung

Die Erfindung betrifft ein Verfahren zur Herstellung von N-Formyl-aminocarbonsäureestern.

N-Formyl-aminocarbonsäureester sind wichtige Vorstufen zu Herstellung von Heterocyklen wie Oxazolen [Bull. Chem. Soc. Jpn. 1971, 44, 1407-1410], Imidazolen [J. Med. Chem. 1969, 12(5), 804-806], oder Pyrazinen [Chim. Ind. 1988, 70, 70-71].

Von den N-Formyl-aminocarbonsäureestern ist N-Formylalaninbutylester (FAB) und der N-Formylalaninethylester von besonderer industrieller Bedeutung, da beide Verbindungen Vorstufen zur Herstellung von Vitamin B₆ darstellen [Bull. Chem. Soc. Jpn. 1971, 44, 1407-1410]. Großtechnisch wird beispielsweise FAB in mehreren 1000 Tonnen hergestellt.

Die industrielle Synthese geht dabei von Alanin aus, man bildet mit HCl das Hydrochlorid, setzt dann im Salzsauren mit Butanol zum Ester um und formyliert anschließend in einem weiteren Schritt mit Formamid. Die Synthese zum FAB ausgehend von Alanin gelingt in ca. 90% Ausbeute. Nachteilig dabei sind Korrosionsprobleme durch Chlorwasserstoff-Gas bzw. der Anfall von einem Äquivalent Ammoniumchlorid als Nebenprodukt. Außerdem bilden sich industriell bedenkliche Nebenprodukte, wie Alkylchloride und Dialkylether.

Neben der Formylierung mit Formamid werden in der Literatur noch zahlreiche andere Formylierungs-Reagenzien beschrieben, wie beispielsweise Ameisensäure [Bull. Chem. Soc. Jpn. 1972, 45, 1917-1918], gemischte Anhydrid aus Essigsäure und Ameisensäure [Bull. Chem. Soc. Jpn. 1965, 38, 244-246], Orthoameisensäureester [Synthesis 1994, 1023-1025] oder Cyanomethylformiat [Synthesis 1996,1,37-38].

Sämtliche vorstehend beschriebenen Fälle gehen aber vom Hydrochlorid des Alaninesters aus und ein kostengünstiges, chlorfreies Verfahren ist dann nicht möglich. Ferner sind diese Syntheseverfahren aufwendig, da sie über mehrere Stufen laufen.

Eine salzfreie, einstufige Synthese von N-Formyl-aminocarbonsäureestern ausgehend von der Aminosäure ist in Bull. Chem. Soc. Jap. 1972, 45, 1917-1918 beschrieben. Hierbei wird die Aminosäure in Gegenwart von Ameisensäure und eines Alkohols auf Temperaturen von 120 bis 180 °C in einem Autoclav erhitzt. Je nach Substitutionsmuster und verwendeten Alkohol werden dabei Ausbeuten von 35-71% erreicht. Diese Ausbeuten sind für eine großtechnische Anwendung noch nicht befriedigend. Das Verfahren weist ferner den Nachteil auf, daß große Mengen Carbonsäureester als Abfallprodukt anfallen.

Der vorliegenden Erfindung liegt daher die Aufgabe zu Grunde, ein weiteres, salzfreies, einstufiges Verfahren zur Herstellung von N-Formyl-aminocarbonsäureestern zur Verfügung zu stellen, daß die Nachteile des Standes der Technik nicht aufweist, großtechnisch anwendbar ist und die N-Formyl-aminocarbonsäureester in hohen Ausbeuten, Selektivitäten und mit geringen Nebenproduktmengen liefert.

Diese Aufgabe wurde erfindungsgemäß gelöst durch ein Verfahren zur Herstellung von N-Formyl-aminocarbonsäureestern, indem man Aminocarbonsäuren mit Ameisensäureestern umsetzt.

Unter Aminocarbonsäuren werden in an sich bekannter Weise organische Verbindungen verstanden die eine freie Aminofunktion und eine freie Carbonsäurefunktion tragen. Das erfindungsgemäße Verfahren ist nicht auf bestimmte Aminocarbonsäuren beschränkt und daher auf alle Aminocarbonsäuren anwendbar.

Vorzugsweise verwendet man als Aminocarbonsäuren Aminocarbonsäuren, ausgewählt aus der Gruppe der Verbindungen der Formel III und IV, 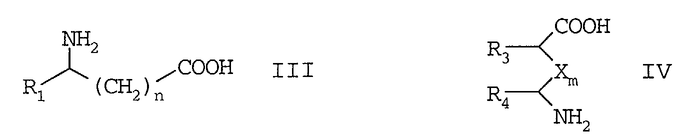 wobei
- n: 0 bis 12,
- m: 0 bis 4,
- R₁: Wasserstoff, einen verzweigten oder unverzweigten, gegebenenfalls substituierten C₁-C₁₂-Alkyl-, C₂-C₁₂-Alkenyl-, C₂-C₁₂-Alkinyl- oder C₁-C₆-Alkylen-C₃-C₇-Cycloalkylrest, einen gegebenenfalls substituierten C₃-C₇-Cycloalkyl-, Aryl-, Arylalkyl-, Hetaryl- oder Hetarylalkylrest,
- R₃, R₄: beide Reste R₃ und R₄ zusammen einen 5 bis 7-gliedrigen, gegebenenfalls substituierten, gesättigten, ungesättigten oder aromatischen Carbo- oder Heterocyclus, der bis zu drei Heteroatome ausgewählt aus der Gruppe O, N oder S enthalten kann,
- X: (C-R₅₎ oder (CH-R₅) und
- R₅: unabhängig voneinander Wasserstoff, Halogen, -NO_{2,} oder -CN
bedeuten.

Unter Ameisensäurestern werden in an sich bekannter Weise Ester der Ameisensäure mit Alkoholen verstanden. Das erfindungsgemäße Verfahren ist nicht auf bestimmte Ameisensäureester beschränkt und daher auf alle Ameisensäureester anwendbar. Vorzugsweise wird der Ameisensäureester als Edukt in isolierter Form eingesetzt.

Vorzugsweise verwendet man als Ameisensäureester Ameisensäureester der Formel V 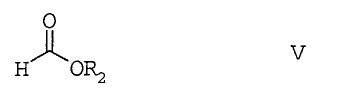 wobei
- R₂: einen verzweigten oder unverzweigten, gegebenenfalls substituierten C₁-C₁₂-Alkyl-, C₂-C₁₂-Alkenyl- oder C₂-C₁₂-Alkinylrest oder einen gegebenenfalls substituierten Aryl- oder Arylalkylrest
bedeutet.

Unter gegebenenfalls substituierten Resten werden erfindungsgemäß die entsprechenden unsubstituierten und substituierten Reste verstanden. Für alle substituierten Reste der vorliegenden Erfindung kommen, wenn die Substituenten nicht näher spezifiziert sind, unabhängig voneinander bis zu 5 Substituenten, beispielsweise ausgewählt aus der folgenden Gruppe in Frage:
-NO_{2,} -OH, -CN, Halogen, einen verzweigten oder unverzweigten, gegebenenfalls substituierten C₁-C₄-Alkylrest,
wie beispielsweise Methyl, CF₃, C₂F₅ oder CH₂F,
einen verzweigten oder unverzweigten, gegebenenfalls substituierten -CO-O-C₁-C₄-Alkyl-, C₃-C₇-Cycloalkyl-, C₁-C₄-Alkoxy-,
C₁-C₄-Thioalkyl-, -NH-CO-O-C₁-C₄-Alkyl, -O-CH₂-COO-C₁-C₄-Alkyl, -NH-CO-C₁-C₄-Alkyl, -CO-NH-C₁-C₄-Alkyl, -NH-SO₂-C₁-C₄-Alkyl, -SO₂-NH-C₁-C₄-Alkyl, -N(C₁-C₄-Alkyl)₂, -NH-C₁-C₄-Alkyl-, oder -SO₂-C₁-C₄-Alkylrest, wie beispielsweise -SO₂-CF₃,
einen gegebenfalls substituierten -NH-CO-Aryl-, -CO-NH-Aryl-, -NH-CO-O-Aryl-, -NH-CO-O-Alkylen-Aryl-, -NH-SO₂-Aryl-, -SO₂-NH-Aryl-, -CO-NH-Benzyl-, -NH-SO₂-Benzyl- oder -SO₂-NH-Benzylrest.

In einer bevorzugten Ausführungsform bedeutet n gleich 0 bis 4, besonders bevorzugt bedeutet n gleich 0 oder 1. n gleich 0 bedeutet, daß der Carboxyl-Kohlenstoff dem α-Kohlenstoff direkt benachbart ist, wie beispielsweise bei natürlichen Aminosäuren.

In einer weiteren bevorzugten Ausführungsform bedeutet m gleich 0 bis 2.

Verzweigte oder unverzweigte C₁-C₁₂-Alkylreste für R₁ und R₂ sind unabhängig voneinander beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, Heptyl, Octyl, Nonyl, Decyl, Undecyl oder Dodecyl, vorzugsweise verzweigte oder unverzweigte C₁-C₄-Alkylreste wie beispielsweise Methyl, Ethyl, Propyl, 1-Methylethyl, Butyl, 1-Methylpropyl, 2-Methylpropyl oder 1,1-Dimethylethyl, besonders bevorzugt Methyl.

Unter einem verzweigten oder unverzweigten C₂-C₁₂-Alkenylrest werden für R₁ und R₂ unabhängig voneinander beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-2-propenyl sowie die entsprechenden Heptenyle, Octenyle, Nonenyle, Decenyle, Undecenyle und Dodecenyle verstanden.

Unter einem verzweigten oder unverzweigten C₂-C₁₂-Alkinylrest werden für R₁ und R₂ unabhängig voneinander beispielsweise Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise Ethinyl, 2-Propinyl, 2-Butinyl, 1-Methyl-2-propinyl oder 1-Methyl-2-butinyl, sowie die entsprechenden Heptinyle, Octinyle, Noninyle, Decinyle, Undecinyle und Dodecinyle verstanden.

Unter einem C₃-C₇-Cycloalkylrest werden für R₁ beispielsweise Cyclopropyl, Cyclobutyl, Cyclopentyl, Cyclohexyl oder Cycloheptyl verstanden.

Verzweigte oder unverzweigte C₁-C₆-Alkylen-C₃-C₇-Cycloalkylreste setzen sich beispielsweise aus verzweigten oder unverzweigten C₁-C₆-Alkylenresten und den vorstehend erwähnten C₃-C₇-Cycloalkylresten zusammen.

Bevorzugte, gegebenenfalls substituierte Arylreste für R₁ und R₂ sind unabhängig voneinander gegebenenfalls substituiertes Phenyl, 1-Naphthyl oder 2-Naphthyl.

Bevorzugte, gegebenenfalls substituierte Arylalkylreste für R₁ und R₂ sind unabhängig voneinander gegebenenfalls substituiertes Benzyl oder Ethylenphenyl (Homobenzyl).

Unter Hetarylresten für R₁ werden beispielsweise Reste wie 2-Pyridyl, 3-Pyridyl, 4-Pyridyl, 2-Furyl, 3-Furyl, 2-Pyrrolyl, 3-Pyrrolyl, 2-Thienyl, 3-Thienyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl verstanden.

Unter substituierten Hetarylresten für R₁ werden auch anellierte Derivate der vorstehend erwähnten Hetarylreste verstanden, wie beispielsweise Indazol, Indol, Benzothiophen, Benzofuran, Indolin, Benzimidazol, Benzthiazol, Benzoxazol, Chinolin, 2,3-Dihydro-1-benzofuran, Furo[2,3]pyridin, Furo[3,2]pyridin oder Isochinolin.

Unter Hetarlyalkylresten werden für R₁ Reste verstanden, die sich beispielsweise aus C₁-C₆-Alkylenresten und aus den vorstehend beschriebenen Hetarylresten zusammensetzen, wie beispielsweise die Reste -CH₂-2-Pyridyl, -CH₂-3-Pyridyl, -CH₂-4-Pyridyl, -CH₂-2-Thienyl, -CH₂-3-Thienyl, -CH₂-2-Thiazolyl, -CH₂-4-Thiazolyl, CH₂-5-Thiazolyl, -CH₂-CH₂-2-Pyridyl, -CH₂-CH₂-3-Pyridyl, -CH₂-CH₂-4-Pyridyl, -CH₂-CH₂-2-Thienyl, -CH₂-CH₂-3-Thienyl, -CH₂-CH₂-2-Thiazolyl, -CH₂-CH₂-4-Thiazolyl, oder -CH₂-CH₂-5-Thiazolyl.

Bevorzugte Reste für R₁ sind Wasserstoff, gegebenenfalls substituiertes C₁-C₁₂-Alkyl, vorzugsweise C₁-C₆-Alkyl, insbesondere C₁-C₄-Alkyl, sowie gegebenenfalls substituiertes Aryl, vorzugsweise Phenyl.

Besonders bevorzugte Reste für R₁ sind die Seitenketten natürlicher Aminosäuren, insbesondere Wasserstoff und Methyl.

Bevorzugte Reste für R₂ sind gegebenenfalls substituiertes C₁-C₁₂-Alkyl, vorzugsweise C₁-C₆-Alkyl, insbesondere C₁-C₄-Alkyl, sowie gegebenenfalls substituiertes Aryl, vorzugsweise Phenyl.

Besonders bevorzugte Reste für R₂ sind Methyl, Ethyl, n-Propyl, Isopropyl, n-Butyl, iso-Butyl- oder tert-Butyl, insbesondere Methyl, Ethyl und n-Butyl.

Die beiden Reste R₃ und R₄ bilden zusammen über den Rest Xₘ einen 5 bis 7-gliedrigen, gegebenenfalls substituierten, gesättigten, ungesättigten oder aromatischen Carbo- oder Heterocyclus, der bis zu drei Heteroatome ausgewählt aus der Gruppe 0, N oder S enthalten kann.

X bedeutet dabei Sauerstoff, Schwefel, Stickstoff, (C-R₅) oder (CH-R₅), wobei die Rest R₅ unabhängig voneinander Wasserstoff, Halogen, -NO_{2,} oder -CN bedeuten.

Für den Fall X gleich Sauerstoff, Schwefel, Stickstoff bedeutet m gleich 1 oder 2, vorzugsweise 1.

Im Fall X gleich (C-R₅) ist X Teil eines aromatischen oder ungesättigten Cyclus, wobei X an einer Doppelbindung beteiligt ist.

Im Fall X gleich (CH-R₅) ist X Teil eines gesättigten oder ungesättigten Cyclus, wobei X nicht an einer Doppelbindung beteiligt ist.

Beispielsweise können die Reste R₃ und R₄ zusammen über den Rest X (m = 1) oder die Reste X (m>1) einen gegebenenfalls substituierten C₅-C₇-Cycloalkylrest, wie beispielsweise Cyclopentyl, Cyclohexyl oder Cycloheptyl, einen gegebenenfalls substituierten Arylrest, wie beispielsweise Phenyl, 1-Naphthyl oder 2-Naphthyl, einen gegebenenfalls substituierten C₅-C₇-Heterocycloalkylrest, wie beispielsweise gegebenenfalls substituiertes Pyrrolidinyl, Piperazinyl, Morpholinyl, Piperidinyl, Tetrahydrofuranyl, Tetrahydropyranyl, 1,4-Dioxanyl, Hexahydroazepinyl, Oxepanyl, 1,2-Oxathiolanyl oder Oxazolidinyl, einen gegebenenfalls substituierten C₃-C₇-Heterocycloalkenylrest, wie beispielsweise gegebenenfalls substituierte Pyrrolinyle, Oxazolinyle, Azepinyl, Oxepinyl, α-Pyranyl, β-Pyranyl, γ-Pyranyl, Dihydropyranyle, 2,5-Dihydro-pyrrolinyl oder 4,5-Dihydro-oxazolyl, einen gegebenenfalls substituierten Hetarylrest, wie beispielsweise gegebenenfalls substituiertes 2-Pyridyl, 3-Pyridyl, 4-Pyridyl, 2-Furyl, 3-Furyl, 2-Pyrrolyl, 3-Pyrrolyl, 2-Thienyl, 3-Thienyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate wie beispielsweise Indazolyl, Indolyl, Benzothiophenyl, Benzofuranyl, Indolinyl, Benzimidazolyl, Benzthiazolyl, Benzoxazolyl, Chinolinyl oder Isochinolinyl.

In einer bevorzugten Ausführungsform bilden die beiden Reste R₃ und R₄ zusammen über den Rest Xₘ einen 5 bis 7-gliedrigen aromatischen Carbo- oder Heterocyclus, der bis zu drei Heteroatome ausgewählt aus der Gruppe 0, N oder S enthalten kann.

Die besonders bevorzugten Aminocarbonsäuren, ausgewählt aus der Gruppe der Verbindungen der Formel III und IV, setzen sich aus den bevorzugten, vorstehend beschriebenen Resten der Aminocarbonsäuren zusammen. Besonders bevorzugte Aminocarbonsäuren sind die natürlichen Aminosäuren, insbesondere Ala, Arg, Asp, Cys, Phe, Gly, His, Ile, Leu, Met, Glu, Ser, Thr, Val, Trp und Tyr, besonders bevorzugt Alanin.

Die Aminocarbonsäuren können enantiomerenrein, als racemische Gemische oder in beliebigen Stereoisomerenverhältnissen vorliegen.

Die besonders bevorzugten Ameisensäureester der Formel V setzen sich aus dem bevorzugten, vorstehend beschriebenen Rest R₂ zusammen. Ganz besonders bevorzugte Ameisensäureester sind Methylformiat, Ethylformiat und n-Butylformiat.

Dementsprechend betrifft die Erfindung vorzugsweise ein Verfahren zur Herstellung von N-Formyl-aminocarbonsäureester, ausgewählt aus der Gruppe von Verbindungen der Formel I und II 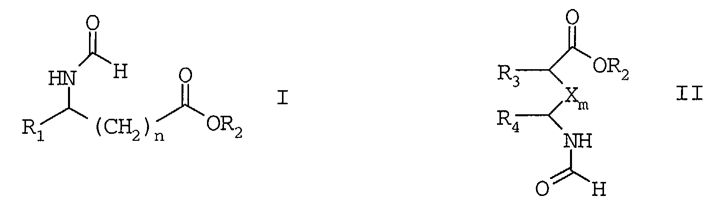 indem man Aminocarbonsäuren, ausgewählt aus der Gruppe der Verbindungen der Formel III und IV 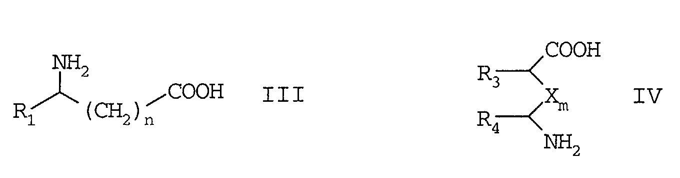 mit Ameisensäureestern der Formel V 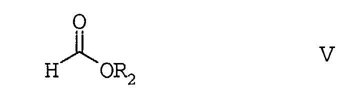 umsetzt, wobei die Reste die vorstehend beschriebene Bedeutung haben.

Bevorzugte N-Formyl-aminocarbonsäureester, ausgewählt aus der Gruppe von Verbindungen der Formel I und II, ergeben sich durch die Verwendung der entsprechenden, bevorzugten Aminocarbonsäuren, ausgewählt aus der Gruppe der Verbindungen der Formel III und IV und der entsprechenden, bevorzugten Ameisensäureester der Formel V als Edukte des erfindunsgemäßen Verfahrens.

Die Temperatur bei der das erfindungsgemäße verfahren durchgeführt wird ist nicht kritisch. Vorteilhafterweise ergeben sich bei Temperaturen größer 110°C höhere Ausbeuten und Selektivitäten. Das Verfahren wird daher vorzugsweise bei 110 bis 200°C, besonders bevorzugt bei 140 bis 180°C, ganz besonders bevorzugt bei 155 bis 165°C durchgeführt.

Der Druck bei dem das erfindungsgemäße verfahren durchgeführt wird ist nicht kritisch. Um bei der Verwendung von Edukten, die unter 110°C sieden, die vorteilhaften Temperaturen von größer 110°C zu erreichen, ist es vorteilhaft das Verfahren unter Eigendruck oder unter einem Druck größer 1 bar durchzuführen. Der Druck übersteigt typischerweise nicht 15 bar.

Besonders vorteilhaft läßt sich das Verfahren in einem Autoclaven durchführen. Hierbei wird das Reaktionsgemisch aus Aminocarbonsäure und Ameisensäureester unter Eigendruck auf die gewünschte Temperatur, vorzugsweise auf die vorstehend beschriebene vorteilhafte Temperatur gebracht.

Das Molverhältnis zwischen Aminocarbonsäure und Ameisensäureester ist ebenfalls nicht kritisch und beträgt vorzugsweise 1:1 bis 1:15, vorzugsweise 1:1 bis 1:10. Ist der Molanteil an Ameisensäureester größer als 1:1 erhält man als Nebenprodukt eine entsprechende Menge Ameisensäureester. Der Ameisensäureester kann als Leichtsieder abdestilliert und in die Reaktion erneut zurückgeführt werden.

In einer weiteren, bevorzugten Ausführungsform verwendet man als Ameisensäureester der Formel V, Mischungen aus Ameisensäureester der Formel V und dem entsprechenden Alkohol R₂-OH. Das Molverhältnis von Ameisensäureester der Formel V zu dem entsprechenden Alkohol R₂-OH ist nicht kritisch und beträgt typischerweise 10:1 bis 1:10, vorzugsweise 1:1 bis 1:5.

Die Reaktionsdauer ist nicht kritisch und beträgt typischerweise 4 bis 24 Stunden, vorzugsweise 6 bis 12 Stunden. Dadurch, dass in jedem Fall die Selektivität der Reaktion über 90% liegt, kann ein Verfahren auch mit Teilumsatz durchgeführt werden.

Der N-Formylaminocarbonsäureester wird üblicherweise destillativ, beispielsweise durch fraktionierte Destillation in an sich bekannter Weise vom Edukt abgetrennt.

Durch das erfindungsgemäße Verfahren werden im Vergleich zum Stand der Technik hohe Umsätze, Ausbeuten und Selektivitäten erreicht.

Die nachstehenden Beispiele verdeutlichen die Erfindung ohne darauf beschränkt zu sein.

### Beispiel 1

In einem Autoclav wurden 13,35 g (0,15 mol) D,L-Alanin in 90,1 g (1,5 mol) Methylformiat suspendiert und unter Eigendruck 12 h bei 160°C unter Stickstoff gerührt. Nichtumgesetztes Alanin wurde abfiltriert und die Lösung fraktioniert destilliert. Man erhielt 18,5 g (0,141 mol) N-Formyl-D,L-alaninmethylester. Dies entspricht einer Ausbeute von 94,2% bei einer Selektivität von 97,5%.

### Beispiel 2

In einem Autoclav wurden 13,35 g (0,15 mol) D,L-Alanin in 111,1 g (1,5 mol) Ethylformiat suspendiert und unter Eigendruck 12 h bei 160°C unter Stickstoff gerührt. Nichtumgesetztes Alanin wurde abfiltriert und die Lösung fraktioniert destilliert. Man erhielt 19,8 g (0,136 mol) N-Formyl-D,L-alaninethylester. Dies entspricht einer Ausbeute von 90,9%.

### Beispiel 3

In einem Autoclav wurden 13,35 g (0,15 mol) D,L-Alanin in 122,4 g (1,2 mol) Butylformiat suspendiert und unter Eigendruck 8 h bei 160°C unter Stickstoff gerührt. Nichtumgesetztes Alanin wurde abfiltriert und die Lösung fraktioniert destilliert. Man erhielt 23,5 g (0,136 mol) N-Formyl-D,L-alaninbutylester (FAB). Dies entspricht einer Ausbeute von 90,4%.

### Beispiel 4

In einem Autoclav wurden 13,35 g (0,15 mol) D,L-Alanin in einem Gemisch aus 85,7 g (0,84 mol) Butylformiat und 26,7 g (0,36 mol) Butanol suspendiert und unter Eigendruck 8 h bei 160°C unter Stickstoff gerührt. Nichtumgesetztes Alanin wurde abfiltriert und die Lösung fraktioniert destilliert. Man erhielt 23,1 g (0,133 mol) N-Formyl-D,L-alaninbutylester (FAB). Dies entspricht einer Ausbeute von 88,8%.

## Patentansprüche

1. Verfahren zur Herstellung von N-Formyl-aminocarbonsäureestern durch Umsetzung von Aminocarbonsäuren mit Ameisensäureestern.

2. Verfahren nach Anspruch 1, **dadurch gekennzeichnet daß** man N-Formyl-aminocarbonsäureester, ausgewählt aus der Gruppe von Verbindungen der Formel I und II 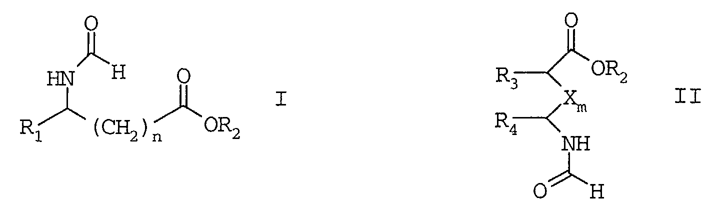 herstellt, wobei
n 0 bis 12,
m 0 bis 4,
R₁ Wasserstoff, einen verzweigten oder unverzweigten, gegebenenfalls substituierten C₁-C₁₂-Alkyl-, C₂-C₁₂-Alkenyl-, C₂-C₁₂-Alkinyl- oder C₁-C₆-Alkylen-C₃-C₇-Cycloalkylrest, einen gegebenenfalls substituierten C₃-C₇-Cycloalkyl-, Aryl-, Arylalkyl-, Hetaryl- oder Hetarylalkylrest,
R₂ einen verzweigten oder unverzweigten, gegebenenfalls substituierten C₁-C₁₂-Alkyl-, C₂-C₁₂-Alkenyl- oder C₂-C₁₂-Alkinylrest, einen gegebenenfalls substituierten Aryl- oder Arylalkylrest,
R₃, R₄ beide Reste R₃ und R₄ zusammen über den Rest Xₘ einen 5 bis 7-gliedrigen, gegebenenfalls substituierten, gesättigten, ungesättigten oder aromatischen Carbo- oder Heterocyclus, der bis zu drei Heteroatome ausgewählt aus der Gruppe 0, N oder S enthalten kann,
X O, S, N, (C-R₅₎ oder (CH-R₅) und
R₅ unabhängig voneinander Wasserstoff, Halogen, -NO_{2,} oder -CN
bedeuten,
indem man Aminocarbonsäuren, ausgewählt aus der Gruppe der Verbindungen der Formel III und IV 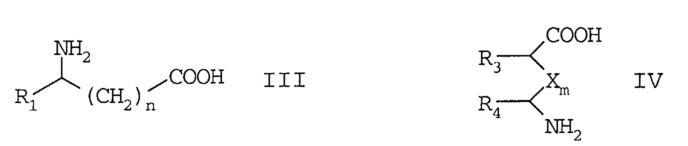 mit Ameisensäureestern der Formel V 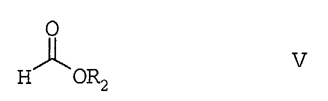 umsetzt.

3. Verfahren nach Anspruch 1 oder 2, **dadurch gekennzeichnet, daß** man das Verfahren bei 110°C bis 200°C durchführt.

4. Verfahren nach einem der Ansprüche 1 bis 3, **dadurch gekennzeichnet, daß** man das Verfahren bei Verwendung von Edukten die unter 110°C sieden unter Eigendruck oder unter einem Druck größer 1 bar durchführt.

5. Verfahren nach einem der Ansprüche 1 bis 4, **dadurch gekennzeichnet, daß** das Molverhältnis zwischen Aminocarbonsäure und Ameisensäureester 1:1 bis 1:10 beträgt.

6. Verfahren nach einem der Ansprüche 2 bis 5 **dadurch gekennzeichnet, daß** man als Ameisensäureester der Formel V, Mischungen aus Ameisensäureester der Formel V und dem entsprechenden Alkohol R₂-OH verwendet.
